(19) Europäisches Patentamt
European Patent Office
Office européen des brevets

(11) Numéro de publication: **0 402 227 A1**

# (12) DEMANDE DE BREVET EUROPEEN

(21) Numéro de dépôt: **90401504.7**

(22) Date de dépôt: **05.06.90**

(51) Int. Cl.5: **C07D 237/22, C07D 237/14, A61K 31/50**

(30) Priorité: **07.06.89 FR 8907532**

(43) Date de publication de la demande: **12.12.90 Bulletin 90/50**

(84) Etats contractants désignés: **AT BE CH DE DK ES FR GB GR IT LI LU NL SE**

(71) Demandeur: **PIERRE FABRE MEDICAMENT**
**125, Rue de la Faisanderie**
**F-75116 Paris(FR)**

(72) Inventeur: **Mouzin, Gilbert**
**11 rue des Pénitents Blancs**
**F-31000 Toulouse(FR)**
Inventeur: **Cousse, Henri**
**La foun de los Nobios, Chemin de Lastinos**
**F-81100 Castres(FR)**
Inventeur: **Patoiseau, Jean-François**
**7 rue Jules Ferry**
**F-81100 Castres(FR)**
Inventeur: **Autin, Jean-Marie**
**Péri-Albo, Viviers les Montagnes**
**F-81290 Labruguiere(FR)**
Inventeur: **Bigg, Denis**
**122 avenue de Lavaur**
**F-81100 Castres(FR)**

(74) Mandataire: **Ahner, Francis et al**
**CABINET REGIMBEAU, 26, avenue Kléber**
**F-75116 Paris(FR)**

## (54) Dérivés de phényl-1-dihydro-1,4 amino-3 oxo-4 pyridazines, leur préparation et leur application en thérapeutique.

(57) La présente invention se rapporte à des dérivés de phényl-1 dihydro-1,4 amino-3 oxo-4 pyridazines répondant à la formule générale I :

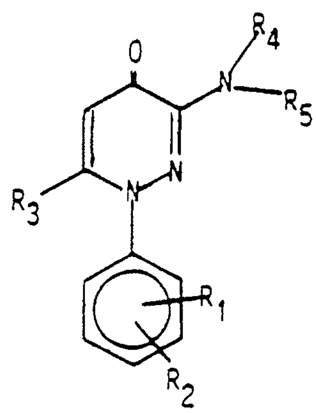

I

Elle concerne également les compositions pharmaceutiques comprenant à titre de principe actif au moins un de ces composés de formule générale I

EP 0 402 227 A1

La présente invention, réalisée au Centre de Recherche PIERRE FABRE MEDICAMENT, a pour objet de nouveaux composés chimiques, leur procédé de préparation et leur application en tant que médicaments. Les nouveaux composés chimiques, revendiqués par la demanderesse répondent à la formule générale I :

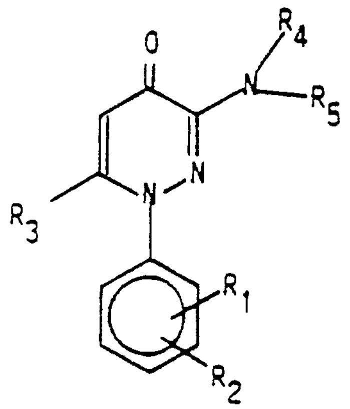

I

dans laquelle :

- $R_1$, $R_2$ identiques ou différents représentent un hydrogène, un groupement alcoyle inférieur ou alcoyloxy, un halogène, un radical trifluorométhyl.
- $R_3$ représente un hydrogène, un groupement alcoyle inférieur en $C_{1-6}$ ramifié ou non, aryle ou arylalcoyle, à l'exception de méthyle lorsque $R_1$, $R_2$, $R_4$ et $R_5$ = H.
- $R_4$ représente l'hydrogène, un radical alcoyle en $C_{1-5}$ linéaire ou ramifié, cyclo alcoyle ou forme avec $R_5$ et l'atome d'azote auquel ils sont liés un hétérocycle tel que pyrrole, pyrrolidine, morpholine, pipéridine, imidazole ou pipérazine éventuellement substituée en position 4.
- $R_5$ représente :

. l'hydrogène, un radical alcoyle en $C_{1-5}$ linéaire ou ramifié,
un groupement aminoalcoyle. II

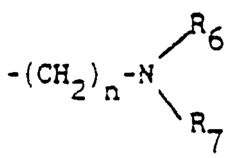

II

dans lequel n est compris entre 1 et 6 et $R_6$, $R_7$ identiques ou différents, représentent l'hydrogène, un groupement alcoyle ou cycloalcoyle ou forment ensemble avec l'atome d'azote auquel ils sont liés, un hétérocycle tel que pipéridine, pyrrolidine, morpholine ou pipérazine éventuellement substituée en position 4 ;

. un groupement $\underset{\underset{O}{\|}}{C}-(O)_m-R_8$ dans lequel

m peut être égal à 0 ou 1 et $R_8$ représente un groupement alcoyle en $C_{1-6}$ linéaire ou ramifié, aryle ou arylalcoyle, amino alcoyle II tel que défini précédemment ;

. un groupement

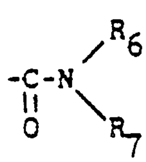

dans lequel $R_6$ et $R_7$ ont la même signification que ci-dessus.

L'invention couvre les sels organiques ou minéraux thérapeutiquement acceptables de ces molécules.

La présente invention concerne également la préparation des dérivés de formule I selon un procédé, caractérisé en ce qu'il comprend :

(a) une étape préliminaire qui consiste à transformer l'acide pyridazine carboxylique de formule III, ou

une de ses formes activées, en son azide et/ou son isocyanate correspondant de formule V

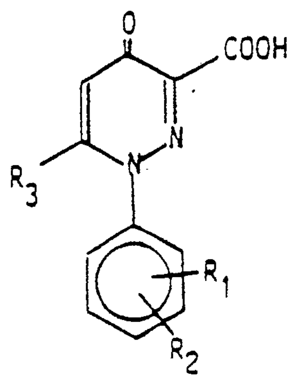

III

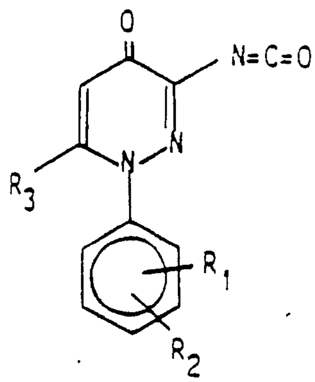

V

$R_1$, $R_2$, $R_3$ ayant la même signification que précédemment

(b) une étape qui consiste à transformer le composé obtenu selon (a) en un composé répondant à la formule générale I et le cas échéant,

(c) une étape pour transformer ce composé de formule I ainsi obtenu en un autre composé de formule I.

Dans l'acide pyridazine carboxylique de formule III, les radicaux $R_1$, $R_2$, $R_3$ ont la même signification que précédemment et qui peut être obtenu selon les méthodes décrites notamment par :

- S. PLESCIA et Coll. J. Heterocyclic. Chem. 18, 333 (1981) $R_3$ = H
- Brevet FR N° 7806494 du 07.03.78
- A. STAEHELIN et Coll. Helv. Chim. Acta 39, 1741 (1956).

Cet acide 555 peut être transformé en isocyanate par dif férentes méthodes :

a) L'acide III traité par le DPPA ou activé sous forme d'un anhydride mixte obtenu par action d'un chloroformate d'alcoyle, puis traité avec un azide tel que l'azoture de Na ou le TMSAzide, fournit après décomposition par chauffage dans un solvant tel que l'acétate Et, le dioxane ou le toluène, l'isocyanate correspondant.

b) L'ester éthylique, obtenu par action du chloroformate d'éthyle en présence de triéthylamine sur l'acide III, est transformé en amide primaire par traitement à l'ammoniaque 30 % dans un solvant tel que le méthanol. L'amide primaire, soumis à l'action d'un hypohalogénure de Na, et plus particulièrement l'eau de Javel dans la soude en présence d'un solvant tel que le THF, permettent d'obtenir l'isocyanate.

L'isocyanate, sans être isolé, peut être traité :

. par une solution aqueuse basique pour fournir l'amine primaire I ($R_4$ = $R_5$ = H) ;

. par un alcool $R_9OH$ pour obtenir le carbamate I ($R_4$ = H, $R_5$ = $\underset{\underset{O}{\|}}{C}-OR_9$) ;

Dans ce cas, l' alcool $R_9OH$ peut être utilisé comme solvant ou cosolvant de réaction pour former l'isocyanate et obtenir le carbamate :

. par une amine

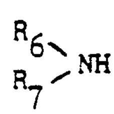

pour obtenir les urées correspondantes I

($R_4$ = H, $R_5$ = 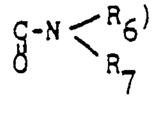)

les radicaux $R_6$ et $R_7$ ayant la même signification que précédemment.

Les amines monosubstituées peuvent être obtenues à partir de l'amine primaire I ($R_4$ = $R_5$ = H) par des techniques classiques telles que benzoylation, alcoylation, débenzoylation. Selon la présente invention,

elles peuvent être également obtenues de façon avantageuse par traitement d'un carbamate par un composé $R_{10}X$ suivi d'une hydrolyse basique, ou acide dans le cas du carbamate de t.butyle.

$R_{10}$ représentant un radical alcoyle linéaire, ramifié ou cyclique, un groupement

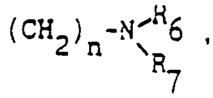

n, $R_6$ et $R_7$ ayant la même signification que précédemment et X représentant un groupe labile tel qu'un halogène.

Les amines tertiaires peuvent être obtenues par alcoylation des amines secondaires par un composé $R_{10}X$, $R_{10}$ et X ayant la même signification que ci-dessus en présence d'un agent tel que l'hydrure de sodium ou la soude.

Selon la présente invention, les amines substituées peuvent également être obtenues par traitement du dérivé chloré IV par une amine

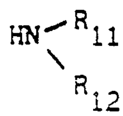

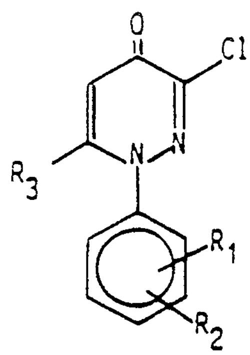

IV

$R_1$, $R_2$, $R_3$ ayant la même signification que précédemment et :

- $R_{11}$ représentant l'hydrogène, un radical alcoyle en $C_{1-5}$ linéaire, ramifié ou cyclique, ou pouvant former avec $R_{12}$ et l'atome d'azote auquel ils sont liés un hétérocycle tel que pyrrole, pyrrolidine, morpholine, pipéridine, imidazole ou pipérazine éventuellement substituée en position 4.
- $R_{12}$ représentant un radical alcoyle en $C_{1-5}$ linéaire ou ramifié ou un groupement

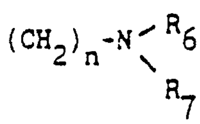

dans lequel $R_6$, $R_7$ et n ont la même signification que précédemment.

Le composé IV peut être obtenu par une réaction de type Sandmeyer en traitant l'amine I ($R_4$ = $R_5$ = H) par un nitrite tel que le nitrite de t.butyle puis le dérivé diazoïque formé par le chlorure cuivrique anhydre. Cette réaction s'effectue dans un milieu anhydre tel que le diméthyl formamide en présence de tamis moléculaires.

Les exemples ci-après illustrent la présente invention.

Les analyses centésimales ainsi que les spectres IR et RMN confirment la structure des composés obtenus.

La présente invention se rapporte également à l'utilisation des intermédiaires de synthèse de formule générale IV à titre de composés nouveaux.

Elle concerne également les compositions pharmaceutiques, caractérisées en ce que à titre de principe

actif,elles contiennent au moins un composé selon l'invention. Ces composés de formule générale I peuvent être, en outre, associés à un autre principe actif dans lesdites compositions.

## Exemple 1

**m.trifluorométhyl phényl-1 dihydro-1,4 oxo-4 amino-3 méthyl-6 pyridazine 1**

Une solution d'acide m.trifluorométhyl phényl-1 dihydro-1,4 oxo-4 méthyl-6 pyridazine-3 carboxylique (29,8 g - 0,1 mole) dans 150 ml d'acétone et 15,5 ml de triéthylamine (0,11 mole) est glacée à -15° C.

Sans dépasser -5° C, on ajoute goutte à goutte 10,5 ml de chloroformate d'éthyle (0,11 mole) puis on agite 2 h à 0° C. On ajoute ensuite une solution d'azoture de sodium (14,6 g - 0,22 mole) dans 60 ml d'eau. L'agitation est maintenue 1 h à 0° C puis l'acétone évaporée sous vide et le résidu repris par 250 ml de toluène.

Après chauffage 1 h à reflux et concentration sous vide, on reprend par 160 ml d'acide chlorhydrique 8H et on chauffe 1 h à 100-110° C.

Le mélange est jeté sur glace, neutralisé à la soude 6N et extrait à l'acétate d'éthyle. Après lavage à l'eau, séchage et concentration, on triture dans l'éther éthylique pour obtenir 19,2 g de composé 1 - (Rendement = 71 %).

F = 178-179° C

CCM : Rf = 0,25 (acétate d'éthyle)

## Exemple 2

**m.trifluorométhyl phényl-1 dihydro-1,4 oxo-4 amino-3 méthyl-6 pyridazine 1**

**a)** m.trifluorométhyl phényl-1 dihydro-1,4 oxo-4 méthyl-6 pyridazine-3 carboxylate d'éthyle.

Glacer une solution d'acide m.trifluorométhyl phényl-1 dihydro-1,4 oxo-4 méthyl-6 pyridazine-3 carboxylique (105 g - 0,35 mole) dans le chloroforme (750 ml) contenant de la triéthylamine (58,5 ml-0,42 mole). Ajouter goutte à goutte le chloroformate d'éthyle (36 ml - 0,376 mole). Après une heure d'agitation à 0° C, laisser revenir à température ambiante, laver au bicarbonate de sodium, à l'eau puis à l'eau salée. Après séchage sur sulfate de sodium, concentrer la phase organique sous vide et reprendre à l'éther éthylique.

On obtient, après filtration, lavage et séchage, 105 g d'ester.

(Rendement = 92 %)

F = 169-170° C

CCM : Rf = 0,24 (acétate d'éthyle).

**b)** m. trifluorométhyl phényl-1 dihydro-1,4 oxo-4 méthyl-6 pyridazine-3 carboxamide.

L'ester obtenu ci-dessus (98,6 g - 0,3 mole) est dissous à 60° C dans le méthanol (750 ml). On ajoute alors l'ammoniaque à 30 % (490 ml) et chauffe une heure à 65-70° C.

Le méthanol est évaporé sous vide. Après reprise à l'eau, filtration, lavage et séchage sous vide, on obtient 87 g de carboxamide.

(Rendement = 96 %).

F = >250° C

CCM : Rf = 0,40 (MeOH-$CHCl_3$ : 15-85)

**c)** m.trifluorométhyl phényl-1 dihydro-1,4 oxo-4 amino-3 méthyl-6 pyridazine 1

A une suspension de 2,97 g (0,01 mole) de carboxamide obtenu ci-dessus, dans le THF (100 ml), on ajoute la soude N (40 ml - 0,04 mole) et l'eau de Javel à 24° (15 ml). Après une heure sous agitation à

température ambiante, la suspension initiale est devenue limpide et le mélange est alors porté à 60 °C pendant 1 heure. Après refroidissement, le mélange est dilué à l'acétate d'éthyle, lavé à l'eau puis séché, filtré et concentré sous vide. Le résidu obtenu est repris à l'éther éthylique, filtré et séché pour obtenir le composé 1 (2,20 g).
(Rendement = 81 %)
F = 179 °C
CCM : Rf = 0,25 (acétate d'éthyle).

## Exemple 3

**m.trifluorométhyl phényl-1 dihydro-1,4 t.butyloxycarbonylamino-3 oxo-4 méthyl-6 pyridazine 2**

**a)** A une suspension d'acide m.trifluorométhyl phényl-1 dihydro-1,4 oxo-4 méthyl-6 pyridazine-3 carboxylique (2,98 g - 0,01 mole) dans 20 ml de terbutanol, on ajoute 1,54 ml (0,011 mole) de triéthylamine et 2,6 ml (0,012 ml) de diphénylphosphorylazide puis chauffe 2 h à 80 °C.

**b)** Le mélange est évaporé sous vide et repris au bicarbonate de sodium, puis extrait à l'acétate d'éthyle. Après lavage à l'eau, séchage sur sulfate de sodium et évaporation sous vide, le composé 2 est recristallisé d'un mélange éther-hexane 50-50 (3,16 g)
(Rendement = 85 %)
F = 170-171 °C
CCM : Rf = 0,42 (acétate d'éthyle)

**c)** Le mélange réactionnel obtenu selon b) est additionné de 16 ml d'acide chlorhydrique 6N, chauffé 30 minutes à 50 °C puis jeté sur 50 ml de soude 2N glacée.

Après extraction à l'acétate d'éthyle, lavage à l'eau, séchage sur sulfate de sodium et évaporation sous vide, le composé 1, tel que défini dans l'exemple précédent, cristallise d'un mélange éther éthylique-hexane 10-90 (2,2 g)
(Rendement = 80 %)
F = 174-175 °C

## Exemple 4

**m. trifluorométhyl phényl-1 dihydro-1,4 oxo-4 amino-3 pyridazine 3**

En opérant de manière identique à celle décrite à l'exemple 2a et 2c, on obtient à partir de l'acide m.trifluorométhyl phényl-1 dihydro-1,4 oxo-4 pyridazine-3 carboxylique, le composé 3.
F = 131 °C
CCM : Rf = 0.3 (acétate d'éthyle).

## Exemple 5

**m.trifluorométhyl phényl-1 dihydro-1,4 (n.butyl 3′) uréido-3 oxo-4 méthyl-6 pyridazine 4**

2,98 g d'acide m.trifluorométhyl phényl-1 dihydro-1,4 oxo-4 méthyl-6 pyridazine-3 carboxylique dans 50 ml d'acétate d'éthyle sont traités pendant 2 h 30 à 60 °C en présence de triéthylamine (2,1 ml) et de diphényl phosphorylazide (3,4 ml). On ajoute ensuite 2,96 g de n.butylamine et maintient à 60 °C pendant 30 mn. Le mélange réactionnel est lavé par une solution de $NaHCO_3$ puis à l'eau, séché sur $Na_2S_5O_4$ et évaporé à sec.

On obtient après trituration dans l'éther éthylique le composé 4 (2,2 g) (Rendement = 60 %)
F = 157 °C
CCM : Rf = 0,7 (méthanol-$CHCl_3$ : 15-85)

## Exemple 6

**m.trifluorométhyl phényl-1 dihydro-1,4 benzamido-3 oxo-4 méthyl-6 pyridazine 5**

A une solution de 8,07 g de composé 1 dans l'acétate d'éthyle (180 ml), on ajoute 6 ml de triéthylamine puis goutte à goutte 4,2 ml de chlorure de benzoyle dans 20 ml d'acétate d'éthyle.

Après 2 h d'agitation, dilution à l'eau, décantation puis lavage à l'eau, la phase organique est séchée sur $Na_2SO_4$ et évaporée sous vide.

On obtient par trituration dans l'éther éthylique et recristallisation dans l'acétate d'éthyle, le composé 5 (8 g)
(Rendement = 71 %)
F = 186° C
CCM : Rf = 0,3 (acétate d'éthyle)

## Exemple 7

**m.trifluorométhyl phényl-1 dihydro-1,4 méthylamino-3 oxo-4 méthyl-6 pyridazine 6**

A une solution de benzamide 5 (5,48 g) dans le diméthylacétamide (50 ml) on ajoute 337 mg de chlorure de benzyl triéthylammonium, 5,85 ml de soude 10N et 1,58 ml de sulfate de méthyle. Après 1 h d'agitation à 20° C, la solution est diluée à l'acétate d'éthyle et à l'eau, décantée, lavée à l'eau puis concentrée sous vide. On reprend par 50 ml de soude N et 50 ml d'éthanol 95° et chauffe 3 heures à reflux. Après reprise à l'acétate d'éthyle, lavage à l'eau et séchage sur $Na_2SO_4$, la phase organique est évaporée sous vide et reprise par un mélange éther-éther de pétrole. On obtient ainsi le composé 6 brut qui peut être recristallisé dans l'acétate d'éthyle (2,48 g)
(Rendement = 59 %)
F = 179° C
CCM : Rf = 0,4 (méthanol-$CHCl_3$ : 15-85)

## Exemple 8

**m.trifluorométhyl phényl-1 dihydro-1,4 diméthylamino-3 oxo-4 méthyl-6 pyridazine 7**

Le composé 6 (3,9 g) est traité par l'hydrure de sodium (413 mg) dans le diméthylacétamide (20 ml) pendant 1 h. On ajoute alors 2,42 ml d'iodure de méthyle et agite 1 h à 20° C puis 1 h à 60° C.

Le mélange est jeté sur de l'eau. Les cristaux obtenus sont filtrés, lavés à l'eau puis repris par de l'acétate d'éthyle. Après séchage, décoloration et évaporation, on obtient par reprise dans le mélange éther éthylique-hexane, le composé 7 qui peut être recristallisé dans un mélange éther éthylique-acétone 95-5 (2 g - Rendement = 50 %)
F = 150° C
CCM : Rf = 0,20 (acétate d'éthyle)

## Exemple 9

**p.chloro phényl-1 dihydro-1,4 méthylamino-3 oxo-4 méthyl-6 pyridazine 8**

A une suspension de 1,57 g de p.chloro phényl-1 dihydro-1,4 t.butyloxy carbonylamino-3 oxo-4 méthyl-6 pyridazine obtenu à partir de l'acide correspondant selon l'exemple **2a** et **2b** dans le THF (60 ml), on ajoute 105 mg de CBTA, 9 ml de soude 6N et 1,74 ml d'iodure de méthyle. Après une nuit à température ambiante, le mélange réactionnel additionné d'eau est extrait à l'acétate d'éthyle. La phase organique séchée sur $Na_2SO_4$ et évaporée sous vide fournit une huile épaisse (1,68 g) qui est reprise dans 3 ml de

méthanol et additionnée de 7,8 ml d'acide chlorhydrique 6N. Après 2 h à température ambiante, le mélange est jeté sur 8 ml de soude 6N et extrait à l'acétate d'éthyle. La phase organique séchée et évaporée sous vide est reprise à l'hexane pour précipiter le composé 8 (916 mg)
(Rendement = 78 %)
F = 214-215 °C
CCM : Rf = 0,23 (acétate d'éthyle)

## Exemple 10

### m.trifluorométhyl phényl-1 dihydro-1,4 chloro-3 oxo-4 méthyl-6 pyridazine 9

A une solution de diméthylformamide maintenue à 0 °C, on ajoute successivement des tamis moléculaires broyés (3 g), du nitrite de terbutyle (5 g) du chlorure cuivrique anhydre (2,95 g) et par petites portions, la m.trifluorométhyl phényl-1 dihydro-1,4 oxo-4 amino-3 méthyl-6 pyridazine 1 (5 g) en solution dans le DMF (10 ml).

Après agitation 30 mn à 0 °C, puis 30 mn à 60 °C et filtration, la solution est reprise à l'eau et extraite à l'acétate d'éthyle. Après séchage sur $Na_2SO_4$ et évaporation sous vide, on obtient par trituration dans l'éther le composé (3,81 g)
(Rendement = 71 %)
F = 190 °C
CCM : Rf = 0,3 (acétate d'éthyle)

## Exemple 11

### m.trifluorométhyl phényl-1 dihydro-1,4 (méthyl-4 pipérazino)-3 oxo-4 méthyl-6 pyridazine 10

1,47 g de composé chloré 9 sont traités pendant 2 h 30 à 100 °C par 7,35 ml de H méthyl pipérazine. Après retour à la température ambiante, l'amine en excès est évaporée sous vide. L'huile résiduelle est reprise par une solution de bicarbonate de sodium et extraite à l'acétate d'éthyle. La phase organique lavée à l'eau, à l'eau salée, est séchée puis concentrée sous vide.

Par trituration dans l'éther de pétrole et recristallisation dans l'éther éthylique, on obtient le composé 10 (1,35 g)
(Rendement = 77 %)
F = 130 °C
CCM : Rf = 0,63 ($CHCl_3$-MeOH : 50-50)

## Exemple 12

### m.trifluorométhyl phényl-1 dihydro-1,4 β aminoéthylamino-3 oxo-4 méthyl-6 pyridazine 11

Le dérivé chloré 9 (1 g) dans l'éthylène diamine (5 ml) est porté à 80 °C pendant 2 h. Après évaporation de l'éthylène diamine, on reprend à l'eau salée et extrait à l'acétate d'éthyle.

La phase organique séchée sur $Na_2SO_4$ est concentrée sous vide et le résidu obtenu chromatographié sur colonne de silice (élution $CHCl_3$-MeOH-$NH_4OH$ : 90-9-1). On obtient ainsi le composé 11 (0,86 g)
(Rendement = 81 %)
F = 130 °C
CCM : Rf = 0,15 ($CHCl_3$-MeOH-$NH_4OH$ : 90-9-1)

Le tableau ci-après résume les principaux produits synthétisés qui illustrent l'invention sans en limiter la portée.

| N° | $R_1$ | $R_2$ | $R_3$ | $R_4$ | $R_5$ | PF°C |
|---|---|---|---|---|---|---|
| 1 | m$CF_3$ | H | $CH_3$ | H | H | 179°C |
| 2 | m$CF_3$ | H | $CH_3$ | H | COOtBu | 170-171°C |
| 3 | m$CF_3$ | H | H | H | H | 131°C |
| 4 | m$CF_3$ | H | $CH_3$ | H | $\underset{O}{\overset{}{C}}NHBu$ | 157°C |
| 5 | m$CF_3$ | H | $CH_3$ | H | CO— 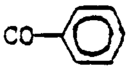 | 186°C |
| 6 | m$CF_3$ | H | $CH_3$ | H | $CH_3$ | 179°C |
| 7 | m$CF_3$ | H | $CH_3$ | $CH_3$ | $CH_3$ | 150°C |
| 8 | pCl | H | $CH_3$ | H | $CH_3$ | 214-215°C |
| 10 | m$CF_3$ | H | $CH_3$ | 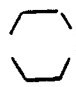 | -$CH_3$ | 130°C |
| 11 | m$CF_3$ | H | $CH_3$ | H | $CH_2CH_2NH_2$ | 130°C |
| 12 | p$OCH_3$ | H | $CH_3$ | H | H | 220°C |
| 13 | O.Cl | H | $CH_3$ | H | H | 215°C |
| 14 | m.Cl | H | $CH_3$ | H | H | 219°C |
| 15 | O.$CF_3$ | H | $CH_3$ | H | H | 236°C |
| 16 | m$CF_3$ | H | $CH_3$ | H | COOEt | 192°C |
| 17 | m$CF_3$ | H | $CH_3$ | H | $COOCH_2$— 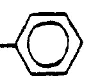 | 151°C |
| 18 | m$CF_3$ | H | $CH_3$ | H | $COCH_3$ | 191°C |
| 19 | m$CF_3$ | H | $CH_3$ | H | $COCH_2NH$— 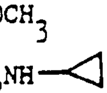 | 220°C |
| 20 | m$CF_3$ | H | $CH_3$ | H | $CH_2CH_2$- 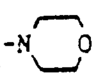 | 101°C |
| 21 | m$CF_3$ | H |  | H | H | 150°C |
| 22 | H | H | $CH_3$ | H | $CH_3$ | 191°C |
| 23 | 2.Cl | 5-$CF_3$ | $CH_3$ | H | H | 107°C |
| 24 | 2.Cl | 5-$CF_3$ | H | H | H | 140°C |
| 25 | m$CF_3$ | H | Et | H | H | 174°C |
| 26 | H | H | $CH_3$ | H | Et | 185°C |
| 27 | m$CF_3$ | H | H | H | $CH_3$ | 126°C |

| | | | | | | |
|---|---|---|---|---|---|---|
| 28 | 2.Cl | 5-Cl | $CH_3$ | H | H | 199°C |
| 29 | 2.Cl | 6-Cl | $CH_3$ | H | H | 227°C |
| 30 | 2.Cl | 5-$CF_3$ | H | H | $CH_3$ | 167°C |
| 31 | m$CF_3$ | H | $CH_3$ | H | 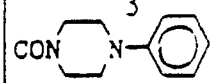 | 163°C |
| 32 | m$CF_3$ | H | $CH_3$ | H | 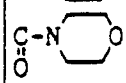 | 236°C |
| 33 | m$CF_3$ | H | $CH_3$ | H | 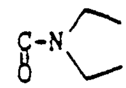 | 139°C |
| 34 | m$CF_3$ | H | $CH_3$ | H | $CONH_2$ | 255°C |
| 35 | m$CF_3$ | H | H | $CH_3$ | $CH_3$ | 130°C |
| 36 | m$CF_3$ | H | $CH_3$ | 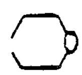 | | 111°C |
| 37 | m$CF_3$ | H | $CH_3$ | 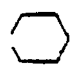 | | 134°C |
| 38 | m$CF_3$ | H | $CH_3$ | 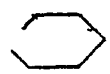 | | 118°C |
| 39 | m$CF_3$ | H | $CH_3$ | 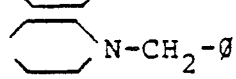 | | 128°C |
| 40 | m$CF_3$ | H | $CH_3$ | 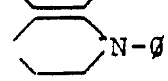 | | 168°C |
| 41 | m$CF_3$ | H | $CH_3$ | H | COOØ | 145°C |
| 42 | m$CF_3$ | H | $CH_3$ | 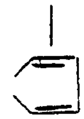 | | 175°C |

## EXPERIMENTATIONS

Divers essais toxicologiques et pharmacologiques ont été effectués sur les composés, objets de la présente invention.

### A - Toxicologie

Les composés de l'invention ont été soumis à des contrôles de toxicité. Celle-ci a été déterminée par la dose létale 50 % (DL 50). Elle a été recherchée sur des lots de 10 souris par voie orale et calculée selon la méthode de Thomson et Weil (Biometrics, 1958, 8, 249).

Les DL 50 des composés testés sont supérieures à 500 mg/kg par voie orale.

### B - Propriétés pharmacologiques

Les expérimentations pharmacologiques ont permis de mettre en évidence de remarquables propriétés sur le système nerveux central et plus particulièrement anxiolytiques.

L'activité anxiolytique des composés de la présente invention a été mise en évidence sur le test de

Vogel (THIEBOT M.H. et al. Eur. J. Pharm. 88, p 111-116, 1983).

Ci-après, sont reportés à titre d'exemple, les résultats obtenus sur certains produits de la présente invention.

| Produit | 30 mg/kg p.o. % d'augment./témoin |
|---|---|
| 1 | 107 % |
| 6 | 64 % |
| 7 | 59 % |
| 16 | 75 % |
| 23 | 141 % |
| 24 | 128 % |
| 27 | 132 % |

## 2) Applications thérapeutiques

Compte tenu de leurs propriétés pharmacologiques, les composés de la présente invention peuvent être utilisés en thérapeutique humaine dans le traitement de diverses maladies mentales et plus particulièrement l'anxiété.

Les préparations pharmaceutiques contenant ces principes actifs peuvent être mises en forme pour l'administration par voie orale, rectale ou parentérale, par exemple capsules, comprimés, gélules, solutés contenant les excipients appropriés.

Il est également possible d'y associer d'autres principes actifs pharmaceutiquement et thérapeutiquement acceptables.

**Revendications**

1 - Composés d'aryl pyridazines correspondant à la formule générale I

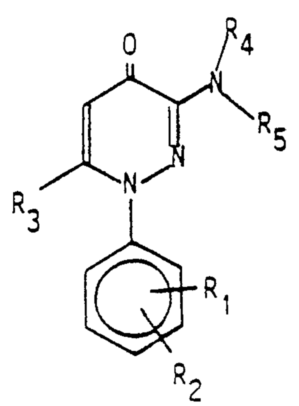

I

dans laquelle :

- $R_1$, $R_2$ identiques ou différents représentent un hydrogène, un groupement alcoyle inférieur ou alcoyloxy, un halogène, un radical trifluorométhyl.
- $R_3$ représente un hydrogène, un groupement alcoyle inférieur en $C_{1-6}$ ramifié ou non, aryle ou arylalcoyle, à l'exception de méthyle lorsque $R_1$, $R_2$, $R_4$ et $R_5$ = H.
- $R_4$ représente l'hydrogène, un radical alcoyle en $C_{1-6}$ linéaire ou ramifié, cyclo alcoyle ou forme avec $R_5$ et l'atome d'azote auquel ils sont liés un hétérocycle tel que pyrrole, pyrrolidine, morpholine, pipéridine, imidazole ou pipérazine éventuellement substituée en position 4.
- $R_5$ représente :

. l'hydrogène, un radical alcoyle en $C_{1-6}$ linéaire ou ramifié,

. un groupement aminoalcoyle II

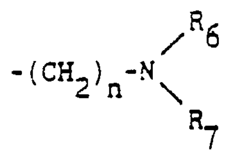

II

dans lequel n est compris entre 1 et 6 et $R_6$, $R_7$ identiques ou différents, représente l'hydrogène, un groupement alcoyle ou cycloalcoyle ou forment ensemble avec l'atome d'azote auquel ils sont liés, un hétérocycle tel que pipéridine, pyrrolidine, morpholine ou pipérazine éventuellement substituée en position 4 ;

. un groupement $\underset{\underset{O}{\|}}{C}\text{-}(O)_m\text{-}R_8$

dans lequel m peut être égal à 0 ou 1 et $R_8$ représente un groupement alcoyle en $C_{1-5}$ linéaire ou ramifié, aryle ou arylalcoyle, amino alcoyle II tel que défini précédemment ;

. un groupement

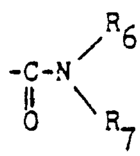

dans lequel $R_6$ et $R_7$ ont la même signification que ci-dessus ; ainsi que les sels organiques ou minéraux thérapeutiquement acceptables de ces molécules.

**2 - Composés de Formule générale 1 selon la revendication 1** caractérisés par le fait qu'ils sont choisis parmi ;

- m.trifluorométhyl phényl-1 dihydro-1,4 oxo-4 amino-3 méthyl-6 pyridazine
- m.trifluorométhyl phényl-1 dihydro-1,4 t.butoxycarbonyl amino-3 oxo-4 méthyl-6 pyridazine
- m.trifluorométhyl phényl-1 dihydro-1,4 oxo-4 amino-3 pyridazine
- m.trifluorométhyl phényl-1 dihydro-1,4 (n butyl 3′) uréido-3 oxo-4 méthyl-6 pyridazine
- m.trifluorométhyl phényl-1 dihydro-1,4 benzamido-3 oxo-4 méthyl-6 pyridazine
- m.trifluorométhyl phényl-1 dihydro-1,4 méthylamino-3 oxo-4 méthyl-6 pyridazine
- m.trifluorométhyl phényl-1 dihydro-1,4 diméthyl amino-3 oxo-4 méthyl-6 pyridazine
- p.chlorophényl-1 dihydro-1,4 méthylamino-3 oxo-4 méthyl-6 pyridazine
- m.trifluorométhyl phényl-1 dihydro-1,4 (méthyl-4 pipérazino)-3 oxo-4 méthyl-6 pyridazine
- m.trifluorométhyl phényl-1 dihydro-1,4 β aminoéthyl amino-3 oxo-4 méthyl-6 pyridazine
- p.méthoxyphényl-1 dihydro-1,4 oxo-4 amino-3 méthyl-6 pyridazine
- o.chlorophényl-1 dihydro-1,4 oxo-4 amino-3 méthyl-6 pyridazine
- m.chlorophényl-1 dihydro-1,4 oxo-4 amino-3 méthyl-6 pyridazine
- o.trifluorométhyl phényl-1 dihydro-1,4 oxo-4 amino-3 méthyl-6 pyridazine
- m.trifluorométhyl phényl-1 dihydro-1,4 oxo-4 méthyl-6 pyridazine-3 carbamate d'éthyle
- m.trifluorométhyl phényl-1 dihydro-1,4 oxo-4 méthyl-6 pyridazine-3 carbamate de benzyle
- m.trifluorométhyl phényl-1 dihydro-1,4 acétamido-3 oxo-4 méthyl-6 pyridazine
- m.trifluorométhyl phényl-1 dihydro-1,4 cyclopropyl glycinamido-3 oxo-4 méthyl-6 pyridazine
- m.trifluorométhyl phényl-1 dihydro-1,4 β morpholino éthylamino-3 oxo-4 méthyl-6 pyridazine
- m.trifluorométhyl phényl-1 dihydro-1,4 amino-3 oxo-4 phényl-6 pyridazine
- phényl-1 dihydro-1,4 méthylamino-3 oxo-4 méthyl-6 pyridazine
- (chloro-2 trifluorométhyl-5) phényl-1 dihydro-1,4 amino-3 oxo-4 méthyl-6 pyridazine
- (chloro-2 trifluorométhyl-5) phényl-1 dihydro-1,4 amino-3 oxo-4 pyridazine
- m.trifluorométhyl phényl-1 dihydro-1,4 amino-3 oxo-4 éthyl-6 pyridazine
- phényl-1 dihydro-1,4 éthylamino-3 oxo-4 méthyl-6 pyridazine
- m.trifluorométhyl phényl-1 dihydro-1,4 méthylamino-3 oxo-4 pyridazine
- (dichloro 2,5 phényl)-1 dihydro-1,4 amino-3 oxo-4 méthyl-6 pyridazine
- (dichloro 2-6 phényl)-1 dihydro-1,4 amino-3 oxo-4 méthyl-6 pyridazine
- (chloro-2 trifluorométhyl-5) phényl-1 dihydro-1 ,4 méthylamino-3 oxo-4 pyridazine
- m. trifluorométhyl phényl-1 dihydro-1,4 oxo-4 méthyl-6 [ (pyrimidinyl 2″) -4′ pipérazino carbonylamino]-3

pyridazine
- m.trifluorométhyl phényl-1 dihydro-1,4 morpholino carbonylamino-3 méthyl-6 pyridazine
- m.trifluorométhyl phényl-1 dihydro-1,4 oxo-4 (diéthyl 3′ 3′) uréido-3 méthyl-6 pyridazine
- m.trifluorométhyl phényl-1 dihydro-1,4 uréido-3 oxo-4 méthyl-6 pyridazine
- m.trifluorométhyl phényl-1 dihydro-1,4 diméthylamino-3 oxo-4 pyridazine
- m.trifluorométhyl phényl-1 dihydro-1,4 morpholino-3 oxo-4 méthyl-6 pyridazine
- m.trifluorométhyl phényl-1 dihydro-1,4 pipéridino-3 oxo-4 méthyl-6 pyridazine
- m.trifluorométhyl phényl-1 dihydro-1,4 pyrrolidino-3 oxo-4 méthyl-6 pyridazine
- m.trifluorométhyl phényl-1 dihydro-1,4 (benzyl-4 pipérazin-1yl)-3 oxo-4 méthyl-6 pyridazine
- m.trifluorométhyl phényl-1 dihydro-1,4 (phényl-4 piperazin-1yl)-3 oxo-4 méthyl-6 pyridazine
- m.trifluorométhyl phényl-1 dihydro-1,4 oxo-4 méthyl-6 pyridazine-3 carbamate de phényle
- m. trifluorométhyl phényl-1 dihydro-1,4 pyrrolo-3 oxo-4 méthyl-6 pyridazine

**3)** Procédé de préparation de composés selon la revendication 1 ou 2, caractérisé en ce qu'il comprend :

(a) une étape préliminaire qui consiste à transformer l'acide pyridazine carboxylique de formule III, ou une de ses formes activées, en son azide et/ou son isocyanate correspondant de formule V :

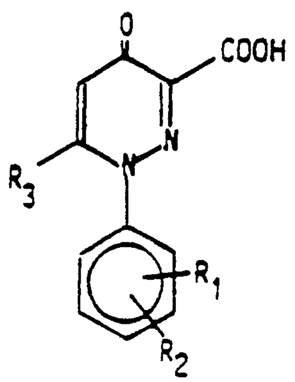

III

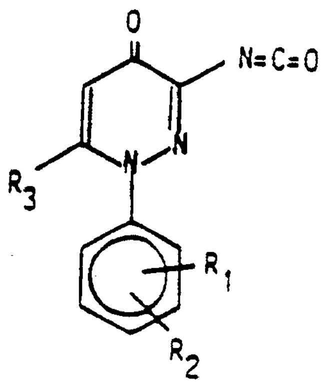

V

**$R_1$, $R_2$, $R_3$ ayant la même signification que précédemment**

(b) une étape qui consiste à transformer le composé obtenu selon (a) en un composé répondant à la formule générale I et le cas échéant,

(c) une étape pour transformer ce composé de formule I ainsi obtenu en un autre composé de formule I.

**4)** Procédé de préparation selon la revendication 3, caractérisé en ce que la forme activée de l'acide est un anhydride mixte obtenu par action d'un chloroformiate d'alcoyle tel que le chloroformiate d'éthyle en présence d'une amine tertiaire telle que la triéthylamine dans un solvant organique tel que l'acétone à une température comprise entre -20° C et +5° C.

**5)** Procédé selon la revendication 3 ou 4, caractérisé en ce que dans l'étape (a), l'isocyanate peut être obtenu en traitant l'acide III ou une de ses formes activées par un azoture et en décomposant thermiquement l'azide formé.

**6)** Procédé selon la revendication 5, caractérisé en ce que l'acide ou sa forme activée non isolée est transformée en azide par action d'un azoture tel que l'azoture de sodium, le diphényl phosphoryl azide ou le triméthyl sylyl azide à une température comprise entre -20° C et +20° C et de préférence à 0° C, et en ce que ledit azide est ensuite décomposé en isocyanate par chauffage dans un solvant tel que le toluène à une température comprise entre +40° C et la température d'ébullition du solvant.

**7)** Procédé selon l'une des revendications 1 et 4, caractérisé en ce que l'isocyanate peut être obtenu en traitant l'amide primaire dérivé de l'acide pyridazine carboxylique III par un hypohalogénure de sodium tel que l'hypochlorure de sodium en milieu basique de préférence un solvant tel que le THF ou dioxane en présence de soude.

**8)** Procédé selon l'une des revendications 3 à 7 , caractérisé en ce que les amines primaires de formule générale I ($R_4$ = $R_5$ = H) sont obtenues par hydrolyse de l'isocyanate en milieu basique tel que la soude à une concentration comprise entre 0,1 et 10N et à une température comprise entre 40° C et 100° C.

**9)** Procédé selon l'une des revendications 3 à 7, caractérisé en ce que les urées de formule générale I sont obtenues en traitant l'azide ou l'isocyanate par l'amine correspondante $R_6$ $R_7$ NH ; $R_6$ et $R_7$ ayant la même signification que précédemment.

**10)** Procédé selon l'une des revendications 3 à 7, caractérisé en ce que les carbamates de formule générale 1 sont obtenus en traitant l'azide ou l'isocyanate par l'alcool correspondant $R_9$ OH, $R_9$ ayant la même signification que précédemment.

**11)** Procédé selon la revendication 10, caractérisé en ce que les carbamates peuvent être alcoylés par un halogénure d'alcoyle dans des conditions de transfert de phase en utilisant de préférence un mélange soude/THF et un catalyseur tel que le chlorure de benzyle triéthyl ammonium.

**12)** Procédé selon la revendication 10, caractérisé en ce que les carbamates peuvent être hydroxylés en milieu basique tel que la soude, à une concentration comprise entre 1 et 10N, pour obtenir les amines correspondantes.

**13)** Procédé selon la revendication 10, caractérisé en ce que les carbamates de tertiobutyle peuvent être hydrolysés par une solution acide telle que l'acide chlorhydrique à une concentration comprise entre 1 et 10N pour obtenir les amines correspondantes.

**14)** Procédé de préparation selon la revendication 3, caractérisé en ce que les amines secondaires ou tertiaires de formule I peuvent être obtenues par action d'une amine $R_{11}$ $R_{12}$ NH avec :

- $R_{11}$ représentant l'hydrogène, un radical alcoyle en $C_{1-5}$ linéaire, ramifié ou cyclique, ou pouvant former avec $R_{12}$ et l'atome d'azote auquel ils sont liés un hétérocycle tel que pyrrole, pyrrolidine, morpholine, pipéridine, imidazole ou pipérazine éventuellement substituée en position 4 et,
- $R_{12}$ représentant un radical alcoyle en $C_{1-5}$ linéaire ou ramifié ou un groupement

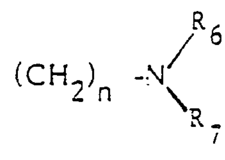

dans lequel $R_6$, $R_7$ et n ont la même signification que précédemment, sur un dérivé chloré de formule IV

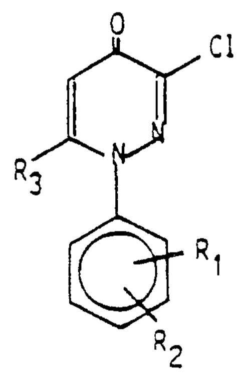

IV

ledit dérivé chloré ayant été obtenu préliminairement à partir d'une amine primaire de formule générale I .

**15)** Procédé de préparation selon la revendication 14, caractérisé en ce que le dérivé chloré IV est obtenu à partir d'une amine primaire de formule générale I par action d'un nitrite tel que le nitrite de t.butyle puis par le chlorure cuivrique anhydre dans un solvant organique anhydre tel que le diméthyl formamide en présence de tamis moléculaires.

**16)** A titre de composés nouveaux, les intermédiaires de synthèse selon la revendication 15 de formule générale IV dans laquelle $R_1$, $R_2$ et $R_3$ ont la même signification que précédemment.

**17)** A titre de médicaments nouveaux, utiles par exemple dans le traitement des troubles du système nerveux central et plus particulièrement comme anxiolytique, les composés définis selon l'une des revendications 1 et 2.

**18)** Compositions pharmaceutiques caractérisées en ce quelles contiennent comme principe actif au moins un composé défini selon l'une des revendications 1 et 2 associé à un support pharmaceutique inerte.

19) Compositions pharmaceutiques selon la revendication 18, caractérisées en ce qu'elles renferment en outre un autre principe actif.

Office européen des brevets

# RAPPORT DE RECHERCHE EUROPEENNE

Numero de la demande

EP 90 40 1504

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 95, 1981, page 677, résumé no. 203879c, Columbus, Ohio, US; G.B. BARLIN: "Reaction of 3,4-diaminopyridazine with alpha,beta-dicarbonyl compounds, carbon disulfide and urea, and methylation of some of the products" * Résumé * ----- | 1 | C 07 D 237/22<br>C 07 D 237/14<br>A 61 K 31/50 |

DOMAINES TECHNIQUES RECHERCHES (Int. Cl.5)

C 07 D 237/00

Le présent rapport a été établi pour toutes les revendications

| Lieu de la recherche | Date d'achèvement de la recherche | Examinateur |
|---|---|---|
| LA HAYE | 10-08-1990 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)